(19) Europäisches Patentamt
European Patent Office
Office européen des brevets

(11) Publication number: 0 240 297
A1

(12) # EUROPEAN PATENT APPLICATION

(21) Application number: 87302765.0

(22) Date of filing: 31.03.87

(51) Int. Cl.[4]: C 07 C 7/20
C 07 C 15/46

(30) Priority: 01.04.86 US 847082 03.02.87 US 10363

(43) Date of publication of application:
07.10.87 Bulletin 87/41

(84) Designated Contracting States:
BE DE FR GB IT NL

(71) Applicant: BETZ EUROPE, INC.
4636 Somerton Road
Trevose Pennsylvania 19047 (US)

(72) Inventor: Perez, Vivian Victoria
351 Kiely Boulevard Apartment B-206
San Jose California 95129 (US)

Martin, John Frederick
522 South Rivershire Drive
Conroe Texas 77304 (US)

Roling, Paul Vincent
4323 Chestergate
Spring Texas 77373 (US)

(74) Representative: Gore, Peter Manson et al
W.P. THOMPSON & CO. Coopers Building Church Street
Liverpool L1 3AB (GB)

(54) **Inhibiting polymerization of vinyl aromatic compounds.**

(57) A method of inhibiting polymerization of a vinyl aromatic compound at elevated temperatures particularly in the preparation of a readily polymerizable vinyl aromatic compound by the use of a substituted hydroxylamine generally having a boiling point higher than the boiling point of the vinyl aromatic compound and a dinitrophenol.

EP 0 240 297 A1

## Description

### INHIBITING POLYMERIZATION OF VINYL AROMATIC COMPOUNDS.

This invention relates to inhibiting polymerization of vinyl aromatic compounds, particularly at elevated temperatures. It especially relates to such inhibition of polymerization in the preparation of the vinyl aromatic compounds.

It is well known in the art that vinyl compounds, specifically vinyl aromatic compounds such as, for example, styrene, readily polymerize and that the rate of polymerization increases with increasing temperature. Common industrial methods for producing the vinyl aromatic compounds typically include separation and purification processes such as distillation to remove impurities. However, purification operations carried out at elevated temperatures result in an increased rate of undesirable polymerization. The polymerization of vinyl aromatic compounds is undesirable because it causes fouling of processing equipment and it renders the compounds unfit for use without further treatment.

Sulphur has been widely employed in the past to inhibit polymerization of vinyl aromatic compounds. However, large quantities of sulphur are required for effective polymerization inhibition and its use results in waste material which presents a significant pollution and/or waste removal problem. More recently, other chemical compounds have been employed as polymerization inhibitors for vinyl aromatic compounds. For example in US-A- 2 965 685 there is disclosed the use of N,N-dialkylhydroxylamine to inhibit polymerization of vinyl aromatic compounds. Also of primary interest is CS-A- 163 428 wherein there is disclosed a method for stabilizing monomers, such as styrene and divinylbenzene, against undesirable polymerization by a radical mechanism by the addition of 2,4-dinitroorthocresol, which may be added in a mixture with 0.0001 to 0.1% by weight of hydroquinone or diethylhydroxylamine, relative to the amount of monomer.

In US-A- 3 148 225 and US-A- 3 371 124 there is disclosed the use of N,N-dialkylhydroxylamine and a mixture of N,N-dialkylhydroxylamine and its bisoxalate salt as popcorn polymer inhibitors in processes relating to the preparation of synthetic rubber. In US-A- 3 390 198 there is disclosed the use of several mono and dialkylcatechols as polymerization inhibitors for hot styrene. In US-A- 3 426 063 there is disclosed the use of N-nitrosoarylhyroxylamine or salt thereof to inhibit thermal polymerization and/or the growth of popcorn polymer in a composition comprising a polymerizable ethylenically unsaturated hydrocarbon and/or ethylenically unsaturated ester. In US-A- 3 849 498 there is disclosed the use of certain dialkylhydroxylamines as polymerization inhibitors for alcoholic solutions of unsaturated aldehydes. US-A- 3 878 181 teaches the use of diethylhydroxylamine either alone or in combination with a water soluble amine to terminate the emulsion polymerization of chloroprene and/or 2,3-dichlorobutadiene.

In US-A- 4 105 506 there is disclosed the use of 2,6-dinitro-p-cresol (DNPC) for polymerization inhibition of vinyl aromatic compounds. Also US-A-4 341 600 teaches the use of N-nitrosodiphenylamine combined with DNPC for inhibiting the polymerization of vinyl toluene under vacuum conditions. In US-A-4 409 408 there is disclosed the use of N,N-dialkylhydroxylamine and tertiary alkylcatechols to inhibit polymerization of vinyl aromatic compounds. Further, US-A- 4 434 307 teaches the use of N,N-diarylhydroxylamine and mono- and di-tertiary alkylcatechols to inhibit polymerization of vinyl aromatic compounds. Also in US-A- 4 439 278 there is disclosed the use of 3,5-dinitrosalicylic acid as a process inhibitor for ethylenically unsaturated aromatic monomer. In US-A- 4 466 905 there is disclosed the use of 4,6-dinitro-p-cresol and either a phenylenediamine or 4-tert-butylcatechol to inhibit polymerization of a vinyl aromatic compound in the presence of oxygen. Additionally, the use of 4,6-dinitro-ortho-cresol alone as a process inhibitor to prevent polymerization of ethylenically unsaturated aromatic compounds is known in the art.

The present invention broadly relates to a method of inhibiting polymerization of a vinyl aromatic compound at elevated temperatures (and also to controlling fouling of processing equipment due to the polymerization) which comprises adding to the vinyl aromatic compound an effective amount for the purpose of a mixture of a substituted hyroxylamine having a boiling point higher than the boiling point of the vinyl aromatic compound, and a dinitrophenol. Preferably, the mixture is comprised of a substituted hydroxylamine selected from N,N-dibenzylhydroxylamine, (methylbenzyl)hydroxylamines and (ethylbenzyl)hydroxylamines and dinitroorthocresol. This mixture provides an unexpectedly higher degree of polymerization inhibition at elevated temperatures than the individual ingredients comprising the mixture. It is therefore possible to produce a more effective polymer inhibiting process at elevated temperatures than is obtainable by the use of either ingredient alone. Because of the enhanced polymer inhibiting activity of the mixture, the total quantity of the polymerization inhibitor required for an effective treatment may be reduced. Additionally, the polymer inhibiting effectiveness which is provided by each of the ingredients may be exploited without use of higher concentrations of each.

Thus, by means of the present invention it is possible to prove methods for inhibiting the polymerization of vinyl aromatic compounds at elevated temperatures and controlling fouling of processing equipment due to the polymerization. It is also possible to provide economically effective polymer inhibiting methods for inhibiting the polymerization of vinyl aromatic compounds such as, for example, styrene. The following detailed description of the invention more particularly demonstrates the synergism of the compounds comprising the present invention.

The substituted hydroxylamines used with a dinitrophenol in accordance with the present invention correspond to the general chemical formula:

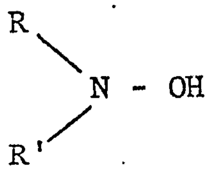

wherein R and R′ are the same of different straight or branched-chain alkyl groups having at least three carbon atoms and preferably having three to about twenty carbon atoms. Alternatively, R or R′ may be a hydrogen with the proviso that R and R′ cannot both be a hydrogen. Also, R and R′ may be the same or different straight or branched-chain aralkyl groups having at least three carbon atoms and preferably having three to about twenty carbon atoms.

The substituted hydroxylamines must also have a boiling point higher than the boiling point of the vinyl aromatic compound utilized in accordance with the present invention. Styrene and other similar vinyl aromatic monomers have boiling points at atmospheric conditions around 140°C and above. Consequently, distillation of these monomers during purification/separation steps in the process of preparation of these vinyl aromatic compounds typically is performed at elevated temperatures of 100 to 140°C under reduced or near atmospheric conditions. Under these conditions, a lower boiling hydroxylamine such as, for example, diethylhydroxylamine (b.p. 125 to 130°C at 101.31 kPa (760 mm Hg)) will go overhead with the monomer vapours resulting in undesirable contamination of the product. Thus, higher boiling hydroxylamines are necessary to prevent such contamination.

Examples of suitable substituted hydroxylamines include N,N-dipropylhydroxylamine, N,N-dibutylhydroxylamine, N,N-2-ethyl-butyloctylhydroxylamine, N,N-didecylhydroxylamine, N,N-dibenzylhydroxylamine, N-benzylhydroxylamine, N,N-butylbenzylhydroxylamine, N-phenylhydroxylamine, N,N-butylphenylhydroxylamine, (methylbenzyl)hydroxylamines, and (ethylbenzyl)hydroxylamines. The term "(methylbenzyl)hydroxylamines" is meant to include mixtures of benzylhydroxylamines and methylbenzylhydroxylamines, such as, for example, Mixture B in Table III described below. Also, the term "(ethylbenzyl)hydroxylamines" is meant to include mixtures of benzylhydroxylamines and ethylbenzylhydroxylamines, such as, for example, Mixture A in Table III described below. Preferably, the substituted hydroxylamine is selected from N,N-dibenzylhydroxylamine, (methylbenzyl)hydroxylamines and (ethylbenzyl)hydroxylamines. It is thought that any dinitrophenol compound may be used in accordance with the present invention. However, dinitroorthocresol is the preferred dinitrophenol compound.

The vinyl aromatic compounds covered in the present invention comprise any of the readily polymerizable vinyl aromatic compounds or monomers including styrene; substituted styrene such as, for example, alpha alkyl styrene, and ring alkyl substituted styrene; diethylenically substituted benzene compounds such as, for example, divinylbenzene, vinyltoluene; vinyl naphthalene and the polyvinylbenzenes. This group is also understood to include all structural isomers and mixtures thereof. The vinyl aromatic compound preferred for use in the present invention is styrene.

The total amount of substituted hydroxylamine and dinitrophenol compound used in the methods of the present invention as a polymerization inhibitor is that amount which is sufficient to effect inhibition of polymerization and will, of course, vary accord ing to the particular vinyl aromatic compound and conditions under which it is used, e.g., temperature, pressure, etc. At higher temperatures, larger amounts are required. Preferably, the total amount of substituted hydroxylamine and dinitrophenol is from about 1 ppm to about 10,000 ppm based upon the weight of the vinyl aromatic compound. Most preferably the total amount of the aforesaid compounds is from about 5 ppm to about 1000 ppm based upon the weight of the vinyl aromatic compound. The relative concentrations of substituted hydroxylamine and dinitrophenol are generally in the range of about 1 to about 99 weight percent substituted hydroxylamine and about 99 to about 1 weight percent dinitrophenol based on the total combined weight of these components. Preferably, the molar ratio of substituted hydroxylamine to dinitrophenol is about 1:1.

The present invention provides an improvement in processes for the preparation of readily polymerizable vinyl aromatic compounds, especially the process for the preparation of styrene, which include a distillation step at elevated temperature. Inasmuch as vinyl aromatic compounds produced by common industrial processes contain impurities, these compounds must be subjected to separation and purification processes in order to be suitable for most types of further industrial use. Such separation and purification are generally accomplished by distillation.

For example, in a typical styrene producing process known in the art, styrene is produced by dehydrogenation of ethylbenzene and so ethylbenzene, toluene, benzene, and styrene will come from a reaction vessel and be passed through three distillation towers:

1. The first distillation tower removes benzene and toluene at about 50°C and 23.3 kPa (about 122°F and 175 mm Hg), while the bottoms product, containing the styrene, is at about 111.7°C and 39.99 kPa (about 233°F and 300 mm Hg).

2. The second distillation tower removes ethylbenzene at about 111.7°C and 39.99 kPa (about 233°F and 300 mm Hg), while the styrene goes bottoms at about 117.8 kPa and 41.32 kPa (about 244°F and 310 mm Hg).

3. In the third distillation tower, the styrene is distilled at about 60°C and 6 kPa (about 140°F and 45 mm Hg), while heavies go bottoms at about 87.8°C and 13.33 kPa (about 190°F and 100 mm Hg).

A fourth distillation tower can be used to remove more styrene monomer from the bottoms product of the third tower.

Dinitroorthocresol is commonly used to prevent polymerization of the styrene during this distillation process. The dinitroorthocresol is added to the crude styrene before the first distillation tower. Since the dinitroorthocresol (b.p. approximately 250°C) has a very high boiling point, it goes bottoms in each step in the process. If the boiling point of a process inhibitor was about the same or lower than the styrene, then the process inhibitor would go overhead and contaminate the purified monomer. Diethylhydroxylamine, while showing synergism with dinitroorthocresol, has a boiling point less than that of styrene and, therefore, the use of diethylhydroxylamine would result in contamination of the purified product (see Table I below).

TABLE I

Boiling Points for Styrene and DEHA

| | Styrene | *Diethylhydroxylamine (DEHA) |
|---|---|---|
| Literature | 145°C/101.31 kPa (293°F/760 mm Hg) | 125-130°C/101.31 kPa (257-266°F/760 mm Hg) |
| Tower 1 | 111.7°C/39.99 kPa (233°F/300 mm Hg) | 93.3°C/39.99 kPa (200°F/300 mm Hg) |
| Tower 2 | 117.8°C/41.32 kPa (244°F/310 mm Hg) | 93.3°C/41.32 kPa (200°F/310 mm Hg) |
| Tower 3 | 59.4°C/6 kPa (139°F/45 mm Hg) | 43.3°C/6 kPa (110°F/45 mm Hg) |

*DEHA tower data projected by use of a nomograph.

Unlike diethylhydroxylamine, the use of the mixture of a higher boiling substituted hydroxylamine and a dinitrophenol in accordance with the present invention controls the fouling of processing equipment, such as, for example, the equipment used in the separation and purification processes of the vinyl aromatic compounds, which is due to or caused by the polymerization of the vinyl aromatic compounds at elevated temperatures. The present invention provides an effective process inhibitor, which is employed during the preparation and processing of the vinyl aromatic compound. Process inhibitors can be distinguished from product inhibitors, which are combined with the vinyl compound in order to inhibit polymerization during storage and handling.

The term "elevated temperatures" as used herein means temperatures at and above the boiling point of the particular vinyl aromatic monomer utilized. Of course, since the boiling points of the various vinyl aromatic monomers are different, the elevated temperatures will vary depending upon the boiling point of the particular vinyl aromatic monomer utilized at the particular pressure at which the mixture is added. For example, the boiling point of styrene is about 145°C at 101.31 kPa (760 mm) pressure, so the elevated temperature for styrene would be 145°C and above at 101.31 kPa (760 mm) pressure. Preferably, the elevated temperatures are part of the distillation conditions comprising a distillation process.

The substituted hydroxylamine and dinitrophenol can be provided to the vinyl aromatic compound by any conventional method. The components can be added to the vinyl aromatic compound as a single mixture containing all of the desired inhibitor compounds or the individual components can be added separately or in any other desired combination. The mixture may be added as either a concentrate or as a solution using a suitable carrier solvent which is compatible with the vinyl aromatic compound being trated. Additionally, the components can be supplied to a vinyl aromatic preparation process in a variety of ways. For example, the

components can be introduced at the beginning of the reaction or they can be added to the apparatus in which the distillation/separation of the crude monomer occurs.

To demonstrate the synergism which is provided by the inventive combination of compounds at elevated temperatures, the data set forth in the Tables associated with the following Examples were developed. The following Examples are included as being illustrations of the present invention and should not be construed as limiting the scope thereof.

EXAMPLES

A closed reflux set-up was used to conduct polymer inhibition test runs. Oxygen contact with the vinyl monomer was minimized by using a bubbler connected to the end of the reflux condenser to avoid back-up flow of oxygen into the set-up. Also, a septum attached to the open neck of a three-necked, round-bottom flask was used to allow easy sampling of the refluxed fluid with a monostat so as to avoid opening the reflux set-up and allowing oxygen contact with the monomer. Nitrogen purging at a low flow rate was conducted throughout the reflux to reduce the presence of oxygen in the system. A controller was used to keep the temperature constant which assisted in keeping the monomer fluid from boiling and overheating.

Samples of 150 mL of monomer were used in all test runs. In runs testing the monomer with treatment compound(s), the treatment was added to the monomer prior to starting the run. Stirring of the monomer was performed using a Teflon-covered magnet, which provided uniform heating of the fluid as it was refluxed since the fluid thickens as polymerization occurs. ("Teflon" is a Trade Mark). A 10-mL aliquot sample was taken in each test run after four hours and six hours of heating at 100°C (212°F). The polymer in the sample was precipitated out of solution by adding methanol in a 2:3 ratio. (refluxed sample:methanol). The polymer was then filtered with a weighed 5.5 cm, No.40 ashless filter paper, and a Buchner filter. The filtered materialwas placed in a desiccator and evacuated for about 24 hours. The filtered material was weighed after it was dried. The results obtained are reported in Table II below.

TABLE II

| RUN | TREATMENT | ppm | After 4 Hours MG* | After 4 Hours % Polymer | After 6 Hours MG | After 6 Hours % Polymer |
|---|---|---|---|---|---|---|
| 1 | NONE | - | 938 | ) | 1591 | ) |
| 2 | NONE | - | 1039 | ) 100 | 1589 | ) 100 |
| 3 | NONE | - | 932 | ) | 1454 | ) |
| 4 | butylated hydroxytoluene (BHT) | 50 | 996 | 100 | 1538 | 100 |
| 5 | 4-tertiary -butylcatchol (TBC) | 152 | 727 | 75 | 1210 | 78 |
| 6 | diethylhydroxylamine (DEHA) | 45 | 341 | 35 | 644 | 42 |
| 7 | dinitro-ortho-cresol (DNOC) | 45 | 272 | 27 | 717 | 47 |
| 8 | DEHA/BHT | 25/25 | 651 | 67 | 945 | 61 |
| 9 | DEHA/TBC | 25/25 | 146 | 15 | 207 | 13 |
| 10 | DEHA/DNOC | 25/25 | 39 | 4 | 78 | 5 |

*MG = milligrams

The results reported in Table II show that the mixture of N,N-diethylhydroxylamine and dinitro-ortho-cresol provides polymerization inhibition. However, at elevated temperatures, the lower boiling diethylhydroxylamine

may contaminate the processed and purified vinyl aromatic compound.

Additional testing was conducted using the insolubles test method to evaluate the synergistic combination of the present invention. In a 17-mL test tube were placed 10.0 mL of double distilled styrene (under vacuum at about 35° C) and the appropriate amounts of inhibitors. The tube was sealed with a septum cap that was wired on. Two needles were placed in the septum cap, and argon was purged through the tube for 30 seconds, whereupon the needles were removed. The tubes were placed in an oil bath regulated at 107.2° C ± 5.4° C for (225° F ± 3° F) for 3.0 hours. At the end of this time, the tubes were cooled in an ice bath and the contents thoroughly mixed with 90 mL of methanol. The precipitated polymer was filtered, dried overnight in an oven, and weighed. All additives were prepared as 2.5 wt.% -solutions in methanol.

Two mixtures were prepared for these additional tests. Mixture A was prepared by placing 15.9 g (0.15 mol) of anhydrous sodium carbonate, 7.0 g (0.1 mol) of hydroxylamine hydrochloride, 11.5 mL (12.5 g, 0.1 mol) of benzyl chloride, 15 mL (16 g, 0.1 mol) of ethylbenzyl chlorides (about 30% ortho and 70% para isomers) and 130 mL of methanol in a 1-L round-bottomed flask. This mixture was heated and stirred at reflux for four hours, whereupon the methanol was removed by distillation. The remaining viscous mixture was stirred with 50 mL of water to yield a clear, colourless aqueous layer and a hazy organic layer. Separation of the layers resulted in 22.8 g (98% of theory) of crude dibenzylhydroxylamines (by gas chromatography - mass spectrometry analysis this mixture consists of N-monobenzylhydroxylamine, N-mono(ethylbenzyl)hydroxylamine, N,N-dibenzylhydroxylamine, N-bnezyl-N-(ethylbenzyl)hydroxylamine, and N,N-di(ethylbenzyl)hydroxylamine, plus other unidentified materials). The starting chlorides were virtually not present in the resulting product. Acidification of the barely basic aqueous layer yielded substantially no $CO_2$. Mixture B was prepared using the same procedure as described above for preparation of Mixture A, but 6.3 (0.05 mol) benzyl chloride. 7.0 g (0.05 mol) of o-methylbenzyl chloride, 7.0 g (0.5 mol) of m-methylbenzyl chloride, and 7.0 g (0.05 mol) of p-methylbenzyl chloride were used in place of the benzyl and ethylbenzyl chlorides, resulting in 20.5 g (87%) yield of N-monobenzylhydroxylamine, N-mono(methylbenzyl)hydroxylamine, N,N-dibenzylhydroxylamine, N-benzyl-N-(methylbenzyl)hydroxylamine, and N,N-dimethylbenzylhydroxylamine, plus other unidentified materials. The results are reported in Table III below.

TABLE III

| Additive | ppm | No. Tests | Grams of Polymer | Ave. Grams of Polymer | Calc. Grams of Polymer |
|---|---|---|---|---|---|
| None | -- | 19 | 1.18, 0.92, 1.10, 1.17, 1.14, 1.31, 0.99, 0.96, 0.96, 0.99, 1.00, 1.16, 0.99, 1.00, 1.02, 0.93, 0.82, 1.00, 1.13 | 1.04 ± 0.12 | - |
| DNOC | 100 | 3 | 0.28, 0.18, 0.20 | 0.22 | - |
| DNOC | 50 | 7 | 0.76, 0.18, 0.64, 0.64, 0.68, 0.31, 0.35 | 0.51 | - |
| DNOC | 25 | 1 | 0.54 | 0.54 | - |
| DNOC | 12.5 | 1 | 1.15 | 1.15 | - |
| DEHA | 100 | 3 | 0.52, 0.33, 0.22 | 0.36 | - |
| DEHA | 12.5 | 1 | 1.15 | 1.15 | - |
| Mixture B | 100 | 1 | 0.84 | 0.84 | - |
| Mixture A-1 | 100 | 2 | 0.62, 0.76 | 0.69 | - |
| Mixture A-2 | 100 | 2 | 0.70, 0.65 | 0.68 | - |
| Mixture A-3 | 100 | 1 | 0.76 | 0.76 | - |
| Mixture A-4 | 100 | 1 | 0.73 | 0.73 | - |
| Mixture A-1 | 50 | 1 | 0.75 | 0.75 | - |
| Mixture A-2 | 50 | 1 | 0.77 | 0.77 | - |
| Mixture A-3 | 50 | 1 | 0.93 | 0.93 | - |

TABLE III (Cont'd)

| Additive | ppm | No. Tests | Grams of Polymer | Ave. Grams of Polymer | Calc. Grams of Polymer |
|---|---|---|---|---|---|
| Mixture A-4 | 50 | 1 | 0.81 | 0.81 | - |
| DEHA/DNOC | 50/50 | 1 | 0.12 | 0.12 | 0.17* |
| Mixture B/DNOC | 50/50 | 1 | 0.19 | 0.19 | 0.41* |
| Mixture A-1/DNOC | 50/50 | 3 | 0.09, 0.08, 0.10 | 0.09 | 0.22** |
| Mixture A-2/DNOC | 50/50 | 2 | 0.45, 0.57 | 0.51 | 0.24** |
| Mixture A-2/DNOC | 50/50 | 1 | 0.08 | 0.08 | 0.24** |
| Mixture A-3/DNOC | 50/50 | 1 | 0.10 | 0.10 | 0.40** |
| Mixture A-4/DNOC | 50/50 | 1 | 0.12 | 0.12 | 0.28** |

*Calculated Grams of Polymer = DNOC at 50 ppm - [(None - other additive at 100 ppm)/2]
= 0.51 - [(1.04 - other additive at 100 ppm)/2]

**Calculated Grams of Polymer = DNOC at 50 ppm - [None - other additive at 50 ppm]
= 0.51 - [1.04 - other additive at 50 ppm]

DNOC = dinitroorthocresol
DEHA = diethylhydroxylamine

The results reported in Table III demonstrate that the higher boiling substituted hydroxylamines are synergistic with dinitroorthocresol in inhibiting polymerization, except for the Mixture A-2-DNOC in which an average of 0.51 grams of polymer resulted -- but it is believed that there may have been a problem with the particular dilute solution of A-2-utilized for dosing the styrene in these tests (i.e., the test runs producing 0.45 and 0.57 grams of polymer). All other test runs with the inventive mixture, including a re-run of A-2 (with a new dilute solution), showed synergism.

## Claims

1. A method of inhibiting polymerization of a vinyl aromatic compound at elevated temperatures which comprises adding to the vinyl aromatic compound an effective amount for the purpose of (a) a substituted hydroxylamine having the general formula:

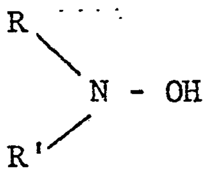

wherein R and R′ are independently hydrogen, alkyl or aralkyl groups, said alkyl or aralkyl groups having at least 3 carbon atoms with the proviso that R and R′ cannot both be hydrogen, the substituted hydroxylamine having a boiling point higher than the boiling point of the vinyl aromatic compound, and (b) a dinitrophenol.

2. A method according to claim 1, wherein the elevated temperatures are part of the distillation conditions comprising a distillation process.

3. A method for the preparation of a readily polymerizable vinyl aromatic compound at elevated temperatures which comprises adding to the vinyl aromatic compound an effective amount for the purpose of (a) a substituted hydroxylamine having the general formula:

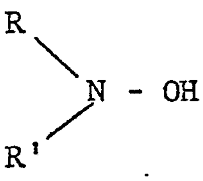

wherein R and R′ are independently hydrogen, alkyl or aralkyl groups, said alkyl or aralkyl groups having at least three carbon atoms, with the proviso that R and R′ cannot both be hydrogen, substituted hydroxylamine having a boiling point higher than the boiling point of the vinyl aromatic compound, and (b) a dinitrophenol.

4. A method according to any of claims 1 to 3, wherein the alkyl of aralkyl groups have three to twenty carbon atoms.

5. A method according to any of claims 1 to 4, wherein the substituted hydroxylamine is selected from N,N-dibenzylhydroxylamine, (methylbenzyl)hydroxylamines and (ethylbenzyl)hydroxylamines.

6. A method according to any of claims 1 to 5, wherein the dinitrophenol is dinitroorthocresol.

7. A method according to any of claims 1 to 6, wherein the total amount of substituted hydroxylamine and dinitrophenol is from about 1 ppm to about 10,000 ppm based upon the weight of the vinyl aromatic compound.

8. A method according to claim 7, wherein the total amount of substituted hydroxylamine and dinitrophenol is from about 5 ppm to about 1000 ppm based upon the weight of the vinyl aromatic compound.

9. A method according to any of claims 1 to 8, wherein the molar ratio of substituted hydroxylamine to dinitrophenol is about 1:1.

10. A method according to any of claims 1 to 9, wherein the vinyl aromatic compound is styrene.

11. A process for the preparation of styrene which includes a distillation step at elevated temperature, which comprises adding to the styrene an effective polymerization inhibiting amount of (a) a substituted hydroxylamine selected from N,N-dibenzylhydroxyl-amine, (methylbenzyl)hydroxylamines and (ethylbenzyl)hydroxylamines, and (b) a dinitroorthocresol.

European Patent Office

# EUROPEAN SEARCH REPORT

Application number

EP 87 30 2765

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | CS-B- 163 428 (D. KONECNY et al.)<br>* Claims *<br>--- | 1,3,6-11 | C 07 C 7/20<br>C 07 C 15/46 |
| A,D | US-A-3 148 225 (H.E. ALBERT)<br>--- | | |
| A,D | US-A-4 466 905 (BUTLER et al.)<br>----- | | |
| | | | TECHNICAL FIELDS SEARCHED (Int. Cl.4) |
| | | | C 07 C 7/00 |

The present search report has been drawn up for all claims

| Place of search | Date of completion of the search | Examiner |
|---|---|---|
| THE HAGUE | 23-06-1987 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82